# EUROPEAN PATENT APPLICATION

(11) **EP 4 621 069 A1**
(43) Date of publication of application: **24.09.2025**
(21) Application number: 23891654.8
(22) Date of filing: 16.11.2023
(51) Int. Cl.: C12Q 1/686, C12N 15/00

(54) **DRY COMPOSITION FOR NUCLEIC ACID AMPLIFICATION USES, AND NUCLEIC ACID AMPLIFICATION METHOD USING SAME**

(30) Priority: 18.11.2022 JP 2022185199
(71) Applicant: NOF Corporation, Shibuya-ku Tokyo 150-6019 (JP)
(72) Inventor: KOHATSU, Haruki, Kawasaki-shi, Kanagawa 210-0865 (JP); SUZUKI, Hirotaka, Kawasaki-shi, Kanagawa 210-0865 (JP); MATSUDA, Masaru, Kawasaki-shi, Kanagawa 210-0865 (JP)
(74) Representative: Weickmann & Weickmann PartmbB
(86) International application number: PCT/JP2023/041267
(87) International publication number: WO 2024/106507

(57) **Abstract**

The present invention provides a dry composition for nucleic acid amplification, containing a polymer containing a constitutional unit derived from a monomer represented by the following formula (1): 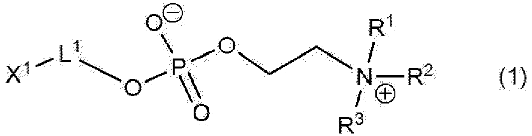 (definitions of symbols in the formula are as described in the specification).

## Description

### [Technical Field]

The present invention relates to a dry composition for nucleic acid amplification and a nucleic acid amplification method using same.

### [Background Art]

Nucleic acid amplification is known as a reaction in which a nucleic acid is used as a template to newly synthesize a nucleic acid complementary to the nucleic acid. In order to performed a nucleic acid amplification reaction, in addition to the nucleic acid to be used as a template, multiple nucleic acid amplification reagents, such as oligonucleotides called primers and enzymes, are required. It is known that these nucleic acid amplification reagents deteriorate significantly at room temperature (e.g., 15°C to 25°C) or under refrigerated conditions due to a decrease in enzyme activity. A method for preventing this deterioration is known to be drying the nucleic acid amplification reagent.

The most generally used drying method is the freeze-drying method (e.g., Patent Literatures 1 to 3). As other drying methods, for example, Patent Literature 4 describes a method of drying at room temperature and atmospheric pressure (air drying method) and a method of drying at room temperature or higher and at a pressure of 90% or less of atmospheric pressure (evaporation drying method).

### [Citation List]

### [Patent Literature]

[Patent Literature 1]
   JP 2000-513940 A
[Patent Literature 2]
   JP H10-503383 A
[Patent Literature 3]
   JP H02-265984 A
[Patent Literature 4]
   JP 2015-092870 A

### [Summary of Invention]

### [Technical Problem]

When a composition for nucleic acid amplification containing a nucleic acid amplification reagent is dried using the drying methods described in Patent Literatures 1 to **4,** the stability of the obtained dry composition for nucleic acid amplification is improved compared to that of the composition solution before drying. However, when a dry composition for nucleic acid amplification is stored for a long period of time, the activity of the nucleic acid amplification reagent decreases due to aggregation, structural changes, adsorption to the container surface, and the like. In other words, even if the drying methods described in Patent Literatures 1 to 4 are used, the storage stability of the obtained dry composition for nucleic acid amplification is still insufficient.

The present invention has been made in consideration of the above problems, and aims to provide a dry composition for nucleic acid amplification superior in storage stability.

### [Solution to Problem]

The present inventors have conducted intensive studies and found that the storage stability of a dry composition for nucleic acid amplification can be improved using a polymer containing a constitutional unit derived from a monomer represented by the following formula (1). Based on this finding, the present invention provides the following.

[1] A dry composition for nucleic acid amplification, comprising a polymer containing a constitutional unit derived from a monomer represented by the formula (1): 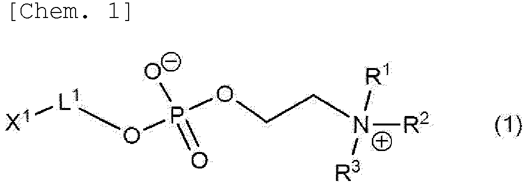 wherein
   X¹ is a (meth)acryloyloxy group or a (meth)acryloylamino group,
   L¹ is an alkylene group having 2 to 4 carbon atoms and optionally having one hydroxy group, or an alkyleneoxyalkylene group having 2 to 4 carbon atoms, and
   R¹ to R³ are each independently an alkyl group having 1 to 3 carbon atoms.
[2] The dry composition for nucleic acid amplification of the aforementioned [1], wherein the aforementioned polymer is a homopolymer consisting of one kind of constitutional unit derived from a monomer represented by the formula (1).
[3] The dry composition for nucleic acid amplification of the aforementioned [1], wherein the aforementioned polymer is a copolymer further comprising a constitutional unit derived from a monomer represented by the formula (2): 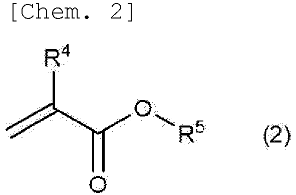 wherein
   R⁴ is a hydrogen atom or a methyl group, and
   R⁵ is a hydrogen atom or an alkyl group having 1 to 20 carbon atoms.
[4] The dry composition for nucleic acid amplification of the aforementioned [1] or [3], wherein the aforementioned polymer is a copolymer further comprising a constitutional unit derived from a monomer represented by the formula (3): 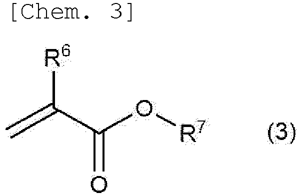 wherein
   R⁶ is a hydrogen atom or a methyl group, and
   R⁷ is an alkyl group having 3 to 6 carbon atoms, and two or more hydroxy groups.
[5] The dry composition for nucleic acid amplification of the aforementioned [1], [3] or [4], wherein the aforementioned polymer is a copolymer further comprising a constitutional unit derived from a monomer represented by the formula (4): 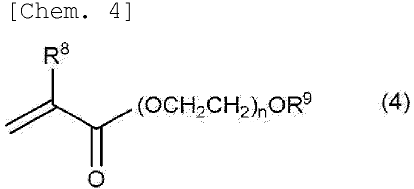 wherein
   R⁸ is a hydrogen atom or a methyl group,
   n is a number from 1 to 10, and
   R⁹ is a hydrogen atom, a methyl group, or an ethyl group.
[6] The dry composition for nucleic acid amplification of any one of the aforementioned [1] to [5], further comprising a polymerase.
[7] The dry composition for nucleic acid amplification of the aforementioned [6], wherein the composition is used for a quantitative polymerase chain reaction method.
[8] The dry composition for nucleic acid amplification of any one of the aforementioned [1] to [7], further comprising a primer.
[9] A method for amplifying a nucleic acid, comprising preparing a reaction liquid by mixing the dry composition for nucleic acid amplification of any one of the aforementioned [1] to [8], a nucleic acid to be amplified, and water, and
   performing a nucleic acid amplification reaction using the obtained reaction liquid.

### [Advantageous Effects of Invention]

According to the present invention, a dry composition for nucleic acid amplification which is superior in storage stability can be obtained.

### [Brief Description of Drawings]

[Fig. 1]

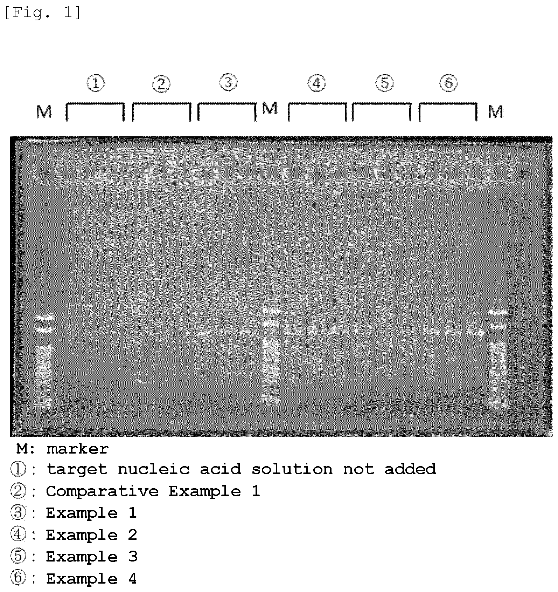

Fig. 1 is a photograph showing the results of electrophoresis performed in Examples 1 to 4 and Comparative Example 1.

### [Description of Embodiments]

The present invention is described in detail below.

When stepwise numerical ranges are described in the present specification, the lower limit and the upper limit of each numerical range can be combined. In the case of, for example, "preferably 10 to 100, more preferably 20 to 90", the preferred lower limit 10 can be combined with the more preferred upper limit 90 (i.e., the numerical range of "10 to 90" is also within the range of the present specification).

### [Dry composition of the present invention]

The present invention provides a dry composition for nucleic acid amplification (sometimes referred to as "dry composition of the present invention" in the present specification) containing a polymer (sometimes referred to as "polymer of the present invention" in the present specification) containing a constitutional unit derived from a monomer represented by the formula (1).

In the present specification, the "dry composition" refers to a solid composition obtained by drying a solution or dispersion.

In the present specification, the "dry composition for nucleic acid amplification" means a dry composition used in the nucleic acid amplification method. More specifically, the "dry composition for nucleic acid amplification" refers to a dry composition for preparing a reaction liquid by mixing the dry composition with a nucleic acid to be amplified and water, and performing a nucleic acid amplification reaction using the obtained reaction liquid.

Examples of the nucleic acid amplification method include Polymerase Chain Reaction (PCR) method, Loop mediated isothermal Amplification (LAMP) method, Transcription Mediated Amplification (TMA) method, Isothermal and Chimeric primer-initiated Amplification of Nucleic acids (IICAN) method, Strand Displacement Amplification (SDA) method, Ligase Chain Reaction (LCR) method, Nucleic Acid Sequence-Based Amplification (NASBA) method, and the like.

The nucleic acid amplification method is preferably a polymerase chain reaction method (PCR method), more preferably a quantitative polymerase chain reaction method. That is, the dry composition of the present invention is more preferably used in the quantitative polymerase chain reaction method. In the present specification, the "polymerase chain reaction method (PCR method)" includes not only a typical PCR method in which DNA is the target nucleic acid, but also a reverse transcription polymerase chain reaction method (RT-PCR method) in which RNA is the target nucleic acid. In the present specification, the "quantitative polymerase chain reaction method" includes not only a typical quantitative polymerase chain reaction method in which DNA is the target nucleic acid, but also a quantitative reverse transcription polymerase chain reaction method in which RNA is the target nucleic acid.

### [Polymer of the present invention]

The polymer of the present invention is polymer containing a constitutional unit derived from a monomer represented by the formula (1): 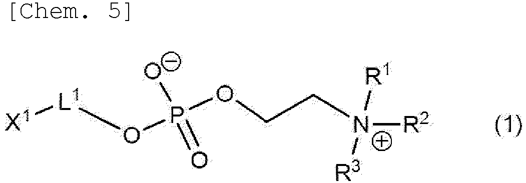 wherein
X¹ is a (meth)acryloyloxy group or a (meth)acryloylamino group,
L¹ is an alkylene group having 2 to 4 carbon atoms and optionally having one hydroxy group, or an alkyleneoxyalkylene group having 2 to 4 carbon atoms, and
R¹ - R³ are each independently an alkyl group having 1 to 3 carbon atoms (hereinafter sometimes to be abbreviated as "monomer (1)"). Only one kind of the polymer of the present invention may be used, or two or more kinds thereof may be used in combination.

The constitutional unit derived from monomer (1) (hereinafter sometimes to be abbreviated as "constitutional unit (1)") means a constitutional unit having a structure formed by the reaction of a carbon-carbon double bond of the (meth)acryloyl group contained in monomer (1). The constitutional units derived from other monomers means the same as the constitutional units derived from monomer (1).

Only one kind of monomer (1) may be used, or two or more kinds thereof may be used in combination. That is, the polymer of the present invention may be a homopolymer consisting of one kind of constitutional unit (1), or a copolymer containing two or more kinds of constitutional units (1). The aforementioned copolymer may be a random copolymer, a block copolymer, or a copolymer containing both a random portion and a block portion.

The groups in the formula (1) are described in order below. In the formula (1), X¹ is a (meth)acryloyloxy group (i.e., CH₂=CR-CO-O-, R: hydrogen atom or a methyl group) or a (meth) acryloylamino group (i.e., CH₂=CR-CO-NH-, R: hydrogen atom or a methyl group). From the aspect of the availability of the starting materials, X¹ is preferably a (meth)acryloyloxy group, more preferably a methacryloyloxy group.

In the formula (1), L¹ is an alkylene group having 2 to 4 carbon atoms and optionally having one hydroxy group, or an alkyleneoxyalkylene group having 2 to 4 carbon atoms. The aforementioned alkylene group may be linear or branched chain. Examples of the alkylene group having 2 to 4 carbon atoms and optionally having one hydroxy group include -C₂H₄-. Examples of the alkyleneoxyalkylene group having 2 to 4 carbon atoms include -C₂H₄-O-C₂H₄-. From the aspect of the availability of the starting materials, L¹ is preferably -C₂H₄- or -C₂H₄-O-C₂H₄-, more preferably -C₂H₄- (i.e., ethylene group).

In the formula (1), R¹ to R³ are each independently an alkyl group having 1 to 3 carbon atoms. The aforementioned alkyl group may be linear or branched chain. Examples of the alkyl group having 1 to 3 carbon atoms include methyl group, ethyl group, propyl group, and the like. From the aspect of the availability of the starting materials, R¹ to R³ are preferably methyl groups.

Preferred monomer (1) is a monomer in which X¹ is a (meth) acryloyloxy group, L¹ is -C₂H₄- or -C₂H₄-O-C₂H₄-, and R¹ to R³ are methyl groups. More preferred monomer (1) is a monomer in which X¹ is a (meth)acryloyloxy group, L¹ is an ethylene group, and R¹ to R³ are methyl groups (i.e., 2-(meth)acryloyloxyethyl phosphorylcholine). Further preferred monomer (1) is 2-methacryloyloxyethyl phosphorylcholine. A commercially available product can be used for monomer (1).

In the present specification, the "2-(meth)acryloyloxyethyl phosphorylcholine" basically means "2-acryloyloxyethyl phosphorylcholine or 2-methacryloyloxyethyl phosphorylcholine". When a plurality of 2-(meth)acryloyloxyethyl phosphorylcholine may be present, the "2-(meth)acryloyloxyethyl phosphorylcholine" means "2-acryloyloxyethyl phosphorylcholine and/or 2-methacryloyloxyethyl phosphorylcholine". Other terms similar to the "2-(meth)acryloyloxyethyl phosphorylcholine" also mean the same as the"(2-(meth)acryloyloxyethyl phosphorylcholine".

One embodiment of the polymer of the present invention is a polymer consisting of constitutional unit (1). In the present specification, the "polymer consisting of constitutional unit (1)" refers to a polymer in which all constitutional units (repeating units) in the polymer chain consist of constitutional unit (1). Expressions similar to the "polymer consisting of constitutional unit (1)" mean the same as the "polymer consisting of constitutional unit (1)". The polymer consisting of constitutional unit (1) may be a homopolymer consisting of one kind of constitutional unit (1) (hereinafter sometimes to be abbreviated as "homopolymer (1)") or a copolymer consisting of two or more kinds of constitutional unit (1). It is preferably homopolymer (1).

While the weight average molecular weight of homopolymer (1) is not particularly limited, it is preferably 20,000 to 2,000,000, more preferably 100,000 to 1,500,000, further preferably 500,000 to 1,500,000. The weight average molecular weight can be determined based on polyethylene glycol by gel permeation chromatography using EcoSEC system (manufactured by Tosoh Corporation) and the like.

The polymer of the present invention may further contain, in addition to constitutional unit (1), a constitutional unit (hereinafter sometimes to be abbreviated as "constitutional unit (2)") derived from a monomer (hereinafter sometimes to be abbreviated as "monomer (2)") represented by the formula (2): 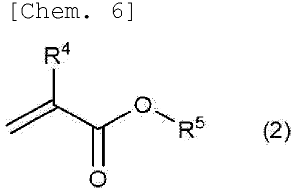 wherein
R⁴ is a hydrogen atom or a methyl group, and
R⁵ is a hydrogen atom or an alkyl group having 1 to 20 carbon atoms.

Only one kind of monomer (2) may be used, or two or more kinds thereof may be used in combination. That is, the polymer of the present invention may be a copolymer containing one or more kinds of constitutional unit (1) and one or more kinds of constitutional unit (2). The aforementioned copolymer may be a random copolymer, a block copolymer, or a copolymer containing both a random portion and a block portion.

The groups in the formula (2) are described in order below. In the formula (2), R⁴ is a hydrogen atom or a methyl group. From the aspect of the storage stability of the polymer, R⁴ is preferably a methyl group.

In the formula (2), R⁵ is a hydrogen atom or an alkyl group having 1 to 20 carbon atoms. The aforementioned alkyl group may be linear or branched chain. It is preferably a hydrogen atom or an alkyl group having 2 to 20 carbon atoms, more preferably a hydrogen atom or an alkyl group having 3 to 19 carbon atoms, further preferably a hydrogen atom or a linear alkyl group having 4 to 18 carbon atoms. When the polymer of the present invention further contains a constitutional unit derived from the monomer represented by the below-mentioned formula (3), in addition to constitutional units (1) and (2), R⁵ is preferably a linear alkyl group having 3 to 6 carbon atoms.

Specific examples of monomer (2) include (meth)acrylic acid, methyl (meth)acrylate, ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, stearyl (meth)acrylate, and the like. A commercially available product can be used for monomer (2).

In the aforementioned specific examples of monomer (2),
(i) (meth)acrylic acid, methyl (meth)acrylate, ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, and stearyl (meth)acrylate are preferred,
(ii) (meth)acrylic acid, methyl (meth)acrylate, ethyl (meth)acrylate, propyl (meth)acrylate, butyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, and stearyl (meth)acrylate are more preferred,
(iii) (meth)acrylic acid, butyl (meth)acrylate, and stearyl (meth)acrylate are further preferred,
(iv) methacrylic acid, butyl methacrylate, and stearyl methacrylate are particularly preferred.

One embodiment of the polymer of the present invention is a copolymer consisting of constitutional units (1) and (2) (hereinafter sometimes abbreviated as "copolymer (1-2)"). In copolymer (1-2), the amount ratio of constitutional unit (1) (i.e., amount ratio of monomer (1) used in polymerization) is preferably 5 to 95 mol, more preferably 10 to 95 mol, further preferably 15 to 95 mol, particularly preferably 20 to 90 mol, and the amount of constitutional unit (2) (i.e., amount ratio of monomer (2) used in polymerization) is preferably 5 to 95 mol, more preferably 5 to 90 mol, further preferably 5 to 85 mol, particularly preferably 10 to 80 mol, each with respect to the total 100 mol of constitutional unit (1) and constitutional unit (2) (i.e., total 100 mol of monomer (1) and monomer (2) used in polymerization).

While the weight average molecular weight of copolymer (1-2) is not particularly limited, it is preferably 10,000 to 2,000,000, more preferably 20,000 to 1,500,000, further preferably 30,000 to 1,000,000.

The polymer of the present invention may further contain, in addition to constitutional unit (1), a constitutional unit (hereinafter sometimes to be abbreviated as "constitutional unit (3)") derived from a monomer (hereinafter sometimes to be abbreviated as "monomer (3)") represented by the formula (3): 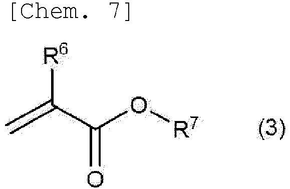 wherein
R⁶ is a hydrogen atom or a methyl group, and
R⁷ is an alkyl group having 3 to 6 carbon atoms and two or more hydroxy groups.

Only one kind of monomer (3) may be used, or two or more kinds thereof may be used in combination. That is, the polymer of the present invention may be a copolymer containing one or more kinds of constitutional unit (1) and one or more kinds of constitutional unit (3), or a copolymer containing one or more kinds of constitutional unit (1), one or more kinds of constitutional unit (2), and one or more kinds of constitutional unit (3). The aforementioned copolymer may be a random copolymer, a block copolymer, or a copolymer containing both a random portion and a block portion.

The groups in the formula (3) are described in order below. R⁶ is a hydrogen atom or a methyl group. From the aspect of the storage stability of the polymer, R⁶ is preferably a methyl group.

In the formula (3), R⁷ is an alkyl group having 3 to 6 carbon atoms, and two or more hydroxy groups. The number of hydroxy groups in R⁷ is preferably 2 to 5. The aforementioned alkyl group may be linear or branched chain. Examples of the alkyl group having 3 to 6 carbon atoms include propyl group, butyl group, pentyl group, hexyl group, and the like.

Specific examples of monomer (3) include, glycerol mono(meth)acrylate, threitol mono(meth)acrylate, erythritol mono(meth)acrylate, xylitol mono(meth)acrylate, arabitol mono(meth)acrylate, mannitol mono(meth)acrylate, galactitol mono(meth)acrylate, sorbitol mono(meth)acrylate, and the like. Among these, glycerol mono(meth)acrylate and xylitol mono(meth)acrylate are preferred, glycerol mono(meth)acrylate is more preferred, and glycerol monomethacrylate is further preferred.

A commercially available product can be used for monomer (3), or may be produced by a known method. For example, monomer (3) can be produced by an esterification reaction of (meth)acrylic acid or a derivative thereof (e.g., acid chloride) with a polyhydric alcohol having 3 or more hydroxy groups. Esterification reaction is well known, those of ordinary skill in the art can set the conditions appropriately and perform the reaction.

One embodiment of the polymer of the present invention is a copolymer consisting of constitutional unit (1) and constitutional unit (3) (hereinafter sometimes abbreviated as "copolymer (1-3)"). In copolymer (1-3), the amount ratio of constitutional unit (1) (i.e., amount ratio of monomer (1) used in polymerization) is preferably 25 to 95 mol, more preferably 30 to 90 mol, further preferably 35 to 85 mol, and the amount of constitutional unit (3) (i.e., amount ratio of monomer (3) used in polymerization) is preferably 5 to 75 mol, more preferably 10 to 70 mol, further preferably 15 to 65 mol, each with respect to the total 100 mol of constitutional unit (1) and constitutional unit (3) (i.e., total 100 mol of monomer (1) and monomer (3) used in polymerization).

While the weight average molecular weight of copolymer (1-3) is not particularly limited, it is preferably 100,000 to 1,000,000, more preferably 100,000 to 800,000, further preferably 100,000 to 500,000.

One embodiment of the polymer of the present invention is a copolymer consisting of constitutional unit (1), constitutional unit (2), and constitutional unit (3) (hereinafter sometimes abbreviated as "copolymer (1-2-3)"). In copolymer (1-2-3), the amount ratio of constitutional unit (1) (i.e., the amount ratio of monomer (1) used in the polymerization) is preferably 30 to 80 mol, more preferably 30 to 70 mol, further preferably 30 to 60 mol, the amount ratio of constitutional unit (2) (i.e., the amount ratio of monomer (2) used in the polymerization) is preferably 10 to 60 mol, more preferably 20 to 60 mol, further preferably 30 to 60 mol, and the amount ratio of constitutional unit (3) (i.e., the amount ratio of monomer (3) used in the polymerization) is preferably 10 to 60 mol, more preferably 10 to 50 mol, further preferably 10 to 40 mol, each with respect to the total 100 mol of constitutional units (1), (2), and (3) (i.e., total 100 mol of monomers (1), (2), and (3) used in the polymerization).

While the weight average molecular weight of copolymer (1-2-3) is not particularly limited, it is preferably 10,000 to 500,000, more preferably 10,000 to 100,000, further preferably 10,000 to 50,000.

The polymer of the present invention may further contain, in addition to constitutional unit (1), a constitutional unit (hereinafter sometimes to be abbreviated as "constitutional unit (4)") derived from a monomer (hereinafter sometimes to be abbreviated as "monomer (4)") represented by the formula (4): 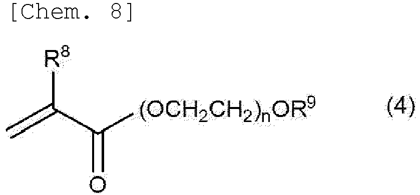 wherein
R⁸ is a hydrogen atom or a methyl group,
n is a number from 1 to 10, and
R⁹ is a hydrogen atom, a methyl group, or an ethyl group.

Only one kind of monomer (4) may be used, or two or more kinds thereof may be used in combination. That is, the polymer of the present invention may be a copolymer containing one or more constitutional units (1) and one or more constitutional units (4), or may be a copolymer containing one or more constitutional units (1), one or more constitutional units (4), and one or more other constitutional units (e.g., one or more constitutional units (2), and/or one or more constitutional units (3)). The aforementioned copolymer may be a random copolymer, a block copolymer, or a copolymer containing both a random portion and a block portion.

The groups in formula (4) are explained below. R⁸ is a hydrogen atom or a methyl group. From the viewpoint of storage stability of the polymer, R⁸ is preferably a methyl group.

n in the formula (4) is a number from 1 to 10, preferably a number from 8 to 10. In addition, n is the number of OCH₂CH₂ units in the polyethylene glycol chain, and n of monomer (4) is practically an average value. Therefore, n may be a decimal number.

R⁹ in formula (4) is a hydrogen atom, a methyl group, or an ethyl group, preferably a methyl group or an ethyl group, more preferably a methyl group.

Specific examples of monomer (4) include polyethylene glycol mono(meth)acrylate, methoxypolyethylene glycol (meth)acrylate, ethoxypolyethylene glycol (meth)acrylate, and the like. From the aspect of the storage stability of the polymer, a methoxypolyethylene glycol (meth)acrylate, an ethoxypolyethylene glycol (meth)acrylate are preferred, and a methoxypolyethylene glycol methacrylate is more preferred.

One embodiment of the polymer of the present invention is a copolymer consisting of constitutional unit (1) and constitutional unit (4) (hereinafter sometimes abbreviated as "copolymer (1-4)"). In copolymer (1-4), the amount ratio of constitutional unit (1) (i.e., the amount ratio of monomer (1) used in the polymerization) is preferably 40 to 95 mol, more preferably 60 to 95 mol, further preferably 80 to 95 mol, and the ratio of constitutional unit (4) (i.e., the amount ratio of monomer (4) used in the polymerization) is preferably 5 to 60 mol, more preferably 5 to 40 mol, further preferably 5 to 20 mol, each with respect to the total 100 mol of constitutional unit (1) and constitutional unit (4) (i.e., total 100 mol of monomers (1) and (4) used in the polymerization).

While the weight average molecular weight of copolymer (1-4) is not particularly limited, it is preferably 100,000 to 1,000,000, more preferably 200,000 to 1,000,000, further preferably 300,000 to 800,000.

The polymer of the present invention may contain, as long as the effect of the present invention is not impaired, other constitutional units derived from other monomers different from the aforementioned monomers (1) to (4). Only one kind of other monomer may be used, or two or more kinds thereof may be used in combination. Other monomer is not particularly limited, and examples thereof include benzyl (meth)acrylate, isobornyl (meth)acrylate, and the like. The amount of other constitutional unit in the polymer of the present invention is preferably not more than 20 mol% with respect to the total constitutional units. More preferably, the polymer of the present invention does not contain other constitutional units.

In one embodiment of the present invention, the polymer of the present invention is
preferably at least one selected from the group consisting of homopolymer (1), copolymer (1-2), copolymer (1-3), copolymer (1-4), and copolymer (1-2-3),
more preferably at least one selected from the group consisting of homopolymer (1), copolymer (1-2), copolymer (1-4), and copolymer (1-2-3),
further preferably homopolymer (1), copolymer (1-2), copolymer (1-3), or copolymer (1-2-3).

Examples of monomers (1) to (4) for forming the constituent units (1) to (4) in this embodiment include those mentioned above.

The polymer of the present invention can be produced by known methods (e.g., the method described in WO 2018/216628).

From the viewpoint of stabilizing effect, the content of the polymer of the present invention relative to the entire dry composition for nucleic acid amplification of the present invention is preferably 0.01% by mass to 10% by mass, more preferably 0.05% by mass to 5% by mass, further preferably 0.1% by mass to 1.0% by mass. When two or more kinds of the polymer of the present invention are used, the aforementioned content means the total content of the two or more kinds of the polymer of the present invention. When two or more kinds of the following other components are used, the contents or amounts described for such other components also refer to the total of the contents or amounts of the two or more components.

### [Other components]

The dry composition of the present invention contains components other than the polymer of the present invention (sometimes referred to as "other components" in the present specification). As other components, known components used for nucleic acid amplification represented by a PCR method can be used. Examples of other component include polymerase, primer, substrate, fluorescent DNA staining reagent, fluorescent probe, passive reference, salt, surfactant, protein, nucleic acid and the like. Only one kind of other component may be used, or two or more kinds thereof may be used in combination.

As the polymerase, known DNA polymerase can be used. From the aspect of heat resistance, enzymes derived from thermophilic bacteria, thermophilic archaea, hyperthermophile, or hyperthermophile archaea, and mutant enzymes thereof are preferred. DNA polymerases are appropriately selected from a DNA-dependent DNA polymerase, an RNA-dependent DNA polymerase, or an enzyme having both functions, depending on the purpose of nucleic acid amplification. Moreover, whether to use a DNA polymerase having nuclease activity or a DNA polymerase having no nuclease activity is appropriately determined.

The dry composition of the present invention preferably contains a polymerase. When the dry composition of the present invention contains a polymerase, the content thereof (i.e., the amount (U) of polymerase per 1 mg of the dry composition) is preferably 0.01 to 3.0 U/mg, more preferably 0.05 to 2.0 U/mg, further preferably 0.1 to 1.0 U/mg.

The primer is not particularly limited. Examples thereof include oligonucleotides with about 15 to 30 bases designed and prepared by known methods. The primer may be modified, as appropriate, with a fluorescent dye such as fluorescein (FAM) and the like. Only one kind of the primer may be used, or two kinds thereof may be used as a pair, or multiple primers may be used to simultaneously amplify multiple regions.

The dry composition of the present invention preferably contains a primer. When the dry composition of the present invention contains a primer, the content thereof (i.e., the amount of primer (pmol) per 1 mg of the dry composition) is preferably 1.0 to 10.0 pmol/mg, more preferably 2.0 to 8.0 pmol/mg, further preferably 3.0 to 6.0 pmol/mg.

While the substrate is not particularly limited, examples thereof include a mixture (dNTPs) of deoxyadenosine triphosphate (dATP), deoxythymidine triphosphate (dTTP), deoxyguanosine triphosphate (dGTP), and deoxycytidine triphosphate (dCTP). It is also possible to partially and/or entirely substitute dTTP with deoxyuridine triphosphate (dUTP).

The fluorescent DNA staining reagent is not particularly limited, and examples thereof include SYBRTM Green I and the like.

The fluorescent probe is not particularly limited, and examples thereof include TaqMan^{™} probe.

The passive reference may be selected appropriately according to the purpose of nucleic acid amplification. Examples of the passive reference include ROX^{™} Dye and the like.

Examples of salt include, but are not limited to, salts of organic bases such as tris(hydroxymethyl)aminomethane, Tricine, Bicine, and the like with acids such as sulfuric acid, hydrochloric acid, acetic acid, phosphoric acid, and the like. In addition, magnesium salts (e.g., magnesium chloride), manganese salts (e.g., manganese acetate), potassium chloride, and ammonium sulfate may also be used as the salt.

Examples of surfactant include, but are not limited to, polyoxyethylene sorbitan fatty acid esters and polyoxyethylene alkylphenyl ethers.

Examples of protein include, but are not limited to, bovine serum albumin.

As the nucleic acid, in addition to the aforementioned primers and fluorescent probes, any DNA and/or RNA may be used as exogenous control genes. Here, the nucleic acid may be synthesized in vitro, or may be prepared from cells, microorganisms, viruses, or the like by known methods. Here, the cells, microorganisms, viruses, and the like may be those collected from human, animals, or plants in the natural world or environment, or may be those obtained by isolation and culture.

The dry composition of the present invention may further contain oil such as mineral oil and the like; a solid support such as glass bead, magnetic bead, and the like.

A kit-type product in which a plurality of the above-mentioned components are selected and combined, and a master mix (sometimes called a primer mix or premix) type product in which such components are premixed, may also be used.

### [Method for producing the dry composition of the present invention]

The dry composition of the present invention can be produced, for example, by drying a solution containing the polymer of the present invention, other components (particularly, polymerase and/or primer), and water. Examples of drying methods for this include a method of drying at room temperature and atmospheric pressure (air drying method), a method of drying at room temperature or higher and at a pressure of 90% or less of atmospheric pressure (evaporation drying method), and a method of freezing the solution and drying same at a low temperature and under high vacuum (freeze drying method). The drying method is preferably freeze drying.

The solution for producing the dry composition of the present invention may contain a buffer. Buffer is not particularly limited. Examples thereof include buffers with a pH adjusted to 6 to 9, more preferably about 7 to 8, by mixing an organic base such as tris(hydroxymethyl)aminomethane, Tricine, Bicine, and the like and an acid such as sulfuric acid, hydrochloric acid, acetic acid, phosphoric acid, and the like. The buffer desirably contains a magnesium salt (e.g., magnesium chloride) and/or a manganese salt (e.g., manganese acetate) as appropriate. The buffer may further contain a salt such as potassium chloride, ammonium sulfate, and the like. The buffer may further contain a water-soluble organic solvent such as dimethyl sulfoxide, dimethylformamide, formamide, glycerol, and the like. The buffer may further contain a surfactant such as polyoxyethylene sorbitan fatty acid ester, polyoxyethylene alkylphenyl ether, and the like. The buffer may further contain a protein such as bovine serum albumin and the like.

### [Nucleic acid amplification method of the present invention]

The present invention also provides a nucleic acid amplification method including preparing a reaction liquid by mixing the dry composition of the present invention, a nucleic acid to be amplified, and water, and performing a nucleic acid amplification reaction using the obtained reaction liquid. The aforementioned reaction liquid may be produced, for example, by mixing a sample containing the nucleic acid to be amplified and water (i.e., an aqueous solution of nucleic acid) with the dry composition of the present invention.

The nucleic acid to be amplified may be either DNA or RNA.

The nucleic acid amplification method is as described above. The nucleic acid amplification method of the present invention is preferably a polymerase chain reaction method (PCR method), more preferably a quantitative polymerase chain reaction method. The nucleic acid amplification method (particularly the PCR method) is well known to those skilled in the art, and can be performed appropriately by those skilled in the art.

### [Example]

The present invention is explained in more detail in the following by referring to Examples and the like, which are not to be construed as limitative.

### [Synthetic Example 1]

2-Methacryloyloxyethylphosphorylcholine (hereinafter sometimes to be indicated as "MPC") (40.0 g) as monomer (1) was weighed into a glass flask for polymerization, purified water (60.0 g) was added to dissolve monomer (1), and Perroyl SA (manufactured by NOF CORPORATION, hereinafter to be referred to as "PRSA") (0.31 g) was added as a polymerization initiator to the obtained solution. After the inside of the reaction container was sufficiently replaced with nitrogen, polymerization was performed by heating at 70°C for 6 hr while stirring. The obtained reaction solution was ice-cooled and added dropwise to acetone to precipitate a polymer. The precipitate was collected by filtration, washed with acetone, and vacuum dried to give a homopolymer (hereinafter to be indicated as "polymer 1") as a white powder. The weight average molecular weight of polymer 1 was 1,030,000 based on polyethylene glycol as measured by gel filtration chromatography (hereinafter to be indicated as "GPC") under the below-mentioned conditions.

### [Synthetic Example 2]

8.4 g of MPC as monomer (1), 2.1 g of butyl methacrylate (hereinafter referred to as "BMA") as monomer (2), and 4.5 g of glycerin monomethacrylate (hereinafter referred to as "GLM") as monomer (3) (monomer (1)/monomer (2)/monomer (3) = 40/40/20 (molar ratio)) were weighed into a glass flask for polymerization, 42.5 g of purified water and 42.5 g of ethanol were added to dissolve monomers (1) to (3), and 0.15 g of PRSA was added to the obtained solution. Thereafter, in the same manner as in Synthetic Example 1, a copolymer (hereinafter to be indicated as "polymer 2") was obtained. The weight average molecular weight of polymer 2 was 22,000 based on polyethylene glycol as measured by GPC under the below-mentioned conditions.

### [Synthetic Example 3]

6.0 g of MPC as monomer (1) and 4.0 g of methacrylic acid (hereinafter referred to as "MAc") as monomer (2) (monomer (1)/monomer (2)=30/70 (molar ratio)) were weighed into a glass flask for polymerization, 90.0 g of purified water was added to dissolve the monomers (1) and (2), and 0.78 g of PRSA was added to the obtained solution. Thereafter, in the same manner as in Synthetic Example 1, a copolymer (hereinafter to be indicated as "polymer 3") was obtained. The weight average molecular weight of polymer 3 was 680,000 based on polyethylene glycol as measured by GPC under the below-mentioned conditions.

### [Synthetic Example 4]

25.3 g of MPC as monomer (1) and 4.7 g of methoxypolyethylene glycol methacrylate (Blenmer PME, manufactured by NOF CORPORATION) as monomer (4) (number average molecular weight: about 500, n in formula (4): about 9, hereinafter referred to as "PEGMA") (monomer (1)/monomer (4) = 90/10 (molar ratio)) were weighed into a polymerization glass flask, 70.0 g of purified water was added to dissolve monomers (1) and (4), and 0.23 g of PRSA was added to the obtained solution. Thereafter, in the same manner as in Synthetic Example 1, a copolymer (hereinafter to be indicated as "polymer 4") was obtained. The weight average molecular weight of polymer 4 was 501,000 based on polyethylene glycol as measured by GPC under the below-mentioned conditions.

### [Synthetic Example 5]

6.0 g of MPC as monomer (1) and 3.3 g of stearyl methacrylate (hereinafter referred to as "SMA") as monomer (2) (monomer (1)/monomer (2) = 80/20 (molar ratio)) were weighed into a glass flask for polymerization, 85.0 g of ethanol was added to dissolve monomers (1) and (2), and 0.06 g of azobisisobutyronitrile was added to the obtained solution as a polymerization initiator. Thereafter, in the same manner as in Synthetic Example 1, a copolymer (hereinafter to be indicated as "polymer 5") was obtained. The weight average molecular weight of polymer 5 was 43,000 based on polyethylene glycol as measured by GPC under the below-mentioned conditions.

### <GPC measurement>

GPC measurement of the polymers 1 to 5 obtained in Synthetic Examples 1 to 5 was performed under the following conditions.
GPC system: EcoSEC system (manufactured by Tosoh Corporation)
column: Shodex OHpak SB-802.5HQ (manufactured by Showa Denko K.K.), and SB-806HQ (manufactured by Showa Denko K.K.) connected in tandem
eluent: 20 mM sodium phosphate buffer (pH 7.4)
detector: differential refractive index detector
molecular weight standard: EasiVial PEG/PEO (manufactured by Agilent Technologies)
flow rate: 0.5 mL/min
column temperature: 40°C
sample: obtained polymer diluted with eluent to final concentration of 0.1 wt%
injection volume: 100 µL

The monomers used in Synthetic Examples 1 to 5 and molar ratios thereof, and weight average molecular weights of the obtained polymers are summarized in Table 1.

**[Table 1]**

| | monomer (1) | monomer (2) | monomer (3) | monomer (4) | monomer molar ratio | weight average molecular weight |
|---|---|---|---|---|---|---|
| polymer 1 | MPC | - | - | - | 100/0/0/0 | 1,030,000 |
| polymer 2 | MPC | BMA | GLM | - | 40/40/20/0 | 22,000 |
| polymer 3 | MPC | MAC | - | - | 30/70/0/0 | 680,000 |
| polymer 4 | MPC | - | - | PEGMA | 90/0/0/10 | 501,000 |
| polymer 5 | MPC | SMA | - | - | 80/20/0/0 | 43,000 |

| | | | | | | |
|---|---|---|---|---|---|---|
| monomer molar ratio =monomer (1)/monomer (2)/monomer (3)/monomer (4) MPC: 2-methacryloyloxyethyl phosphorylcholine BMA: butyl methacrylate GLM: glycerol monomethacrylate MAc: methacrylic acid PEGMA: methoxypolyethylene glycol methacrylate SMA: stearyl methacrylate | | | | | | |

### [Examples 1 - 4]

Examples are shown in which the amplification target nucleic acid is a double-stranded DNA and the nucleic acid amplification method is a PCR method.

### <Preparation of solution A>

The components shown in Table 2 were mixed to prepare solution A. The preparation of solution A was performed while cooling with ice.

**[Table 2]**

| component | final concentration | amount added⁽¹⁾ |
|---|---|---|
| Distilled Water, Nuclease-free (manufactured by NIPPON GENE CO., LTD.) | - | 1.74 µL |
| 5x Colorless GoTaq Flexi Buffer (manufactured by Promega) | 1x⁽²⁾ | 4.00 µL |
| Magnesium Chloride Solution, 25 mM | 1.5 mM | 1.20 µL |
| 2 mM dNTPs (manufactured by TOYOBO CO., LTD.) | 0.2 mM | 2.00 µL |
| first primer | 0.6 µM | 2.00 µL |
| second primer | 0.6 µM | 2.00 µL |
| Go Taq MDx Hot Start Polymerase (manufactured by Promega) | 0.5 U | 0.06 µL |
| total | | 13 µL |

| | | |
|---|---|---|
| (1) amount added: amount added per well (2) 1x: 5-fold diluted concentration of Clorless GoTaq Flexi Buffer | | |

### <Preparation of composition for nucleic acid amplification before drying>

The polymers obtained in Synthesis Examples 1 to 4 were added to the obtained solution A to prepare compositions for nucleic acid amplification. To be specific, in Example 1, 2, or 4, 2 µL of an aqueous solution of polymer 1, 2, or 4 with a concentration of 0.5 w/v% was added to each well of solution A to prepare 15 µL of the compositions for nucleic acid amplification (concentration of polymer in each compositions: 0.067 w/v%). In addition, in Example 3, 2 µL of an aqueous solution of polymer 3 with a concentration of 0.05 w/v% was added to each well of solution A to prepare 15 µL of the composition for nucleic acid amplification (concentration of polymer in the composition: 0.0067 w/v%). All of these compositions were prepared while cooling on ice.

### <Preparation of dry composition for nucleic acid amplification>

The obtained composition for nucleic acid amplification was placed in a PCR tube (manufactured by Eppendorf), and the tube was placed in an ultra-low temperature refrigerator (Ultra Low Temperature Freezer manufactured by NIHON FREEZER CO., LTD.) and stored at -80°C for 30 min to freeze the composition. The PCR tube containing the frozen composition was then placed in a freeze dryer ("FDU-2100" manufactured by TOKYO RIKAKIKAI CO., LTD.) to freeze-dry (sublimation-dry) the composition to prepare a dry composition for nucleic acid amplification. The freeze-drying was performed at a reduced pressure of 15 kPa to 20 kPa and at a temperature of 22°C for 24 hr. The content of each polymer in the obtained dry composition was 0.4% by mass, the content of polymerase was 0.2 U/mg, and the content of primer (i.e., the total content of the first and second primers) was 4.8 pmol/mg.

The band intensity of the nucleic acid amplification product was measured using the dry composition immediately after freeze-drying or the dry composition after three months of storage as follows.

### <Storage of the dry composition>

The dry composition was stored as follows. Specifically, after freeze-drying, the PCR tube was removed from the freeze-dryer and immediately sealed with parafilm to prevent the dry composition from absorbing moisture. The tightly sealed PCR tube containing the dry composition was stored for three months at 37°C.

### <Preparation of composition for nucleic acid amplification from dry composition>

15 µL of Distilled Water, Nuclease-free (manufactured by NIPPON GENE CO., LTD.) was added per well to the dry composition immediately after freeze-drying or the dry composition after three months of storage, and the dry composition was dissolved to prepare a composition for nucleic acid amplification. All of these compositions were prepared while cooling on ice.

### <Preparation of PCR reaction liquid>

An aqueous solution of the target nucleic acid with a nucleic acid concentration of 10 ng/µL was added to the composition for nucleic acid amplification in an amount of 5 µL per well to prepare a PCR reaction liquid (concentration of each polymer in the PCR reaction liquid: 0.05 w/v% in Examples 1, 2, and 4, 0.005 w/v% in Example 3). All of the PCR reaction liquids were prepared while cooling on ice.

### <PCR>

PCR was performed using the PCR reaction liquid obtained as described above with a qPCR device ("StepOnePlus^{™} Real-Time PCR System" manufactured by Applied Biosystems). The reaction conditions (temperature program) are as shown in the following Table 3.

**[Table 3]**

| step | reaction conditions | |
|---|---|---|
| 1 | 94°C, 2 min | |
| 2 | 98°C, 45 sec | 30 cycles |
| 3 | 60°C, 30 sec | |
| 4 | 73°C, 1 min 18 sec | |
| 5 | 73°C, 5 min | |

### <Electrophoresis>

The obtained PCR product was electrophoresed, and the DNA amount (band density) was observed as follows. First, 4 µL of 6x Loading Buffer (manufactured by NIPPON GENE CO., LTD.) was added to 20 µL of the obtained PCR product to prepare an electrophoresis sample. A gel for electrophoresis was prepared using Agarose S (manufactured by NIPPON GENE CO., LTD.). 1x TEA (manufactured by NIPPON GENE CO., LTD.) was used as the electrophoresis buffer. A marker (Gene Ladder 100 (0.1-2 kbp), manufactured by NIPPON GENE CO., LTD.) and an electrophoresis sample were added to the prepared gel for electrophoresis at 4 µL/well, and the mixture was electrophoresed at 100 V for 30 min. Thereafter, the sample components were stained using Midori Green Advance (Genetics), then destained using Distilled Water, Nuclease-free (NIPPON GENE CO., LTD.), and the band of the target nucleic acid amplification product (size: 1.3 kbp) was detected using a gel imaging device (Fas-Digi Compact, Nippon Genetics Co., Ltd.). The band intensity obtained using the dry composition immediately after freeze-drying or after three months of storage was calculated using Image J (manufactured by National Institutes of Health (USA)). The band intensity (relative value) obtained using the dry composition after three months of storage, when the band intensity obtained using the dry composition immediately after freeze-drying is set to 100%, is shown in Table 4 below. Fig. 1 also shows the results of electrophoresis performed using the dry compositions of Examples 1 to 4 after three months of storage. Fig. 1 also shows the results of electrophoresis performed using a marker, electrophoresis without the addition of a target nucleic acid solution, and electrophoresis performed using the dry composition of Comparative Example 1 after three months of storage described below.

### [Comparative Example 1]

In Comparative Example 1, the same operations as in Examples 1 to 4 were performed except that in <Preparation of dry composition for nucleic acid amplification>, 2 µL of Distilled Water, Nuclease-free (manufactured by NIPPON GENE CO., LTD.) was used instead of 2 µL of the aqueous solution of the polymer. The band intensity (relative value) obtained using the dry composition after storage for three months, with the band intensity obtained using the dry composition immediately after freeze-drying taken as 100%, is shown in Table 4 below. Also, Fig. 1 shows the result of electrophoresis performed using the dry composition after storage for three months.

**[Table 4]**

| | polymer used | band intensity (relative value) |
|---|---|---|
| Example 1 | polymer 1 | 36% |
| Example 2 | polymer 2 | 44% |
| Example 3 | polymer 3 | 31% |
| Example 4 | polymer 4 | 51% |
| Comparative Example 1 | - | 0 |

| | | |
|---|---|---|
| band intensity (relative value) = band intensity (relative value) obtained using the dry composition after storage for three months when band intensity obtained using the dry composition immediately after freeze-drying is taken as 100% | | |

As shown in Table 4 and Figure 1, no bands were detected in electrophoresis performed using the dry composition of Comparative Example 1, not containing polymers 1 to 4, after storage for three months, whereas bands were detected in electrophoresis performed using the dry compositions of Examples 1 to 4, containing any of polymers 1 to 4, after storage for three months. In particular, in Example 4, the band intensity (relative value) obtained using the dry composition after storage for three months was 51%. These results reveal that the dry composition for nucleic acid amplification of the present invention is superior in storage stability.

### [Examples 5 - 8]

Examples are shown in which the amplification target nucleic acid is a single-stranded RNA and the nucleic acid amplification method is an RT-qPCR method.

### <Preparation of solution B>

The components shown in Table 5 were mixed to prepare solution B. Solution B was prepared while cooling with ice.

**[Table 5]**

| component | final concentration | amount added⁽¹⁾ |
|---|---|---|
| RapiDxFire Lyo-Flex 1-Step RT-qPCR 5X Master Mix (manufactured by BIOSEARCH) | 1x⁽²⁾ | 4.00 µL |
| Fw Primer NIID_2019-nCOV_N_F2 | 0.5 µM | 1.00 µL |
| Rv Primer NIID_2019-nCOV_N_R2 | 0.5 µM | 1.00 µL |
| TaqMan Probe Primer NIID_2019-nCOV_N_P2 | 0.2 µM | 0.80 µL |
| 50x ROX | 0.2 µM | 0.40 µL |
| Distilled Water, Nuclease-free (manufactured by NIPPON GENE CO., LTD.) | - | 5.80 µL |
| total | | 13 µL |

| | | |
|---|---|---|
| (1) amount added: amount added per well (2) 1x: 5-fold diluted concentration of RapiDxFire Lyo-Flex 1-Step RT-qPCR 5X Master Mix | | |

### <Preparation of composition for nucleic acid amplification before drying>

The polymer obtained in Synthesis Examples 1 or 3 to 5 was added to the obtained solution B to prepare a composition for nucleic acid amplification. To be specific, in Example 5, 7, or 8, 2 µL of an aqueous solution of polymer 1, 4, or 5 with a concentration of 0.5 w/v% was added to each well of solution B to prepare 15 µL of the compositions for nucleic acid amplification (concentration of polymer in each compositions: 0.067 w/v%). In addition, in Example 6, 2 µL of an aqueous solution of polymer 3 with a concentration of 0.05 w/v% was added to each well of solution B to prepare 15 µL of the composition for nucleic acid amplification (concentration of polymer in the composition: 0.0067 w/v%). All of these compositions were prepared while cooling on ice.

### <Preparation of dry composition for nucleic acid amplification>

The obtained composition for nucleic acid amplification was placed in a PCR tube (manufactured by Eppendorf), and the tube was placed in an ultra-low temperature refrigerator (Ultra Low Temperature Freezer manufactured by NIHON FREEZER CO., LTD.) and stored at -80°C for 30 min to freeze the composition. The PCR tube containing the frozen composition was then placed in a freeze dryer ("FDU-2100" manufactured by TOKYO RIKAKIKAI CO., LTD.) to freeze-dry (sublimation-dry) the composition to prepare a dry composition for nucleic acid amplification. The freeze-drying was performed at a reduced pressure of 15 kPa to 20 kPa and at a temperature of 22°C for 24 hr. The content of each polymer in the obtained dry composition was 0.4% by mass and the content of primer (i.e., the total content of forward primer and reverse primer) was 4.0 pmol/mg.

The fluorescence intensity of the nucleic acid amplification product was measured using the dry composition immediately after freeze-drying or the dry composition after 12 days of storage as follows.

### <Storage of the dry composition>

The dry composition was stored as follows. Specifically, after freeze-drying, the PCR tube was removed from the freeze-dryer and immediately sealed with parafilm to prevent the dry composition from absorbing moisture. The tightly sealed PCR tube containing the dry composition was stored for 12 days at 60°C.

### <Preparation of composition for nucleic acid amplification from dry composition>

15 µL of Distilled Water, Nuclease-free (manufactured by NIPPON GENE CO., LTD.) was added per well to the dry composition immediately after freeze-drying or the dry composition after 12 days of storage, and the dry composition was dissolved to prepare a composition for nucleic acid amplification. All of these compositions were prepared while cooling on ice.

### <Preparation of RT-qPCR reaction liquid>

An aqueous solution of the target nucleic acid with a nucleic acid concentration of 100 copies/uL was added to the composition for nucleic acid amplification in an amount of 5 µL per well to prepare an RT-qPCR reaction liquid (concentration of each polymer in the RT-qPCR reaction liquid: 0.05 w/v% in Examples 5, 7 and 8, 0.005 w/v% in Example 6). All of the PCR reaction liquids were prepared while cooling on ice.

### <RT-qPCR>

RT-qPCR was performed using the RT-qPCR reaction liquid obtained as described above with a qPCR device ("StepOnePlus^{™} Real-Time PCR System" manufactured by Applied Biosystems), and the endpoint fluorescence intensity of the RT-qPCR reaction liquid was measured. The reaction conditions (temperature program) are as shown in the following Table 6.

**[Table 6]**

| step | reaction conditions | |
|---|---|---|
| 1 | 85°C, 3 min | |
| 2 | 60°C, 15 min | |
| 3 | 95°C, 2 min | |
| 4 | 95°C, 10 sec | 60 cycles |
| 5 | 60°C, 30 sec | |

The fluorescence intensity (relative value) obtained using the dry composition after 12 days of storage, when the fluorescence intensity obtained using the dry composition immediately after freeze-drying is set to 100%, is shown in Table 7 below.

### [Comparative Example 2]

In Comparative Example 2, the same operations as in Examples 5 to 8 were performed except that in <Preparation of dry composition for nucleic acid amplification>, 2 µL of Distilled Water, Nuclease-free (manufactured by Nippon Gene Co., Ltd.) was used instead of 2 µL of the aqueous solution of the polymer. The fluorescence intensity (relative value) obtained using the dry composition after storage for 12 days, with the fluorescence intensity obtained using the dry composition immediately after freeze-drying taken as 100%, is shown in Table 7 below.

**[Table 7]**

| | polymer used | fluorescence intensity (relative value) |
|---|---|---|
| Example 5 | polymer 1 | 54% |
| Example 6 | polymer 3 | 79% |
| Example 7 | polymer 4 | 82% |
| Example 8 | polymer 5 | 66% |
| Comparative Example 2 | - | 52% |

| | | |
|---|---|---|
| fluorescence intensity (relative value) = fluorescence intensity (relative value) obtained using the dry composition after storage for 12 days when band intensity obtained using the dry composition immediately after freeze-drying is taken as 100% | | |

As shown in Table 7, the dry compositions of Examples 5 to 8 containing polymers 1 or 3 to 5 after 12 days of storage had higher fluorescence intensities (relative values) than the dry composition of Comparative Example 2 not containing these polymers. In particular, in Example 7, the fluorescence intensity (relative value) obtained using the dry composition after storage for 12 days was 82%. These results reveal that the dry composition for nucleic acid amplification of the present invention is superior in storage stability.

### [Industrial Applicability]

The dry composition of the present invention is useful for nucleic acid amplification methods (particularly quantitative polymerase chain reaction method) for genetic testing, microbial testing, viral testing, and the like.

This application is based on a patent application No. 2022-185199 filed in Japan, the contents of which are encompassed in full herein.

## Claims

1. A dry composition for nucleic acid amplification, comprising a polymer containing a constitutional unit derived from a monomer represented by the formula (1): 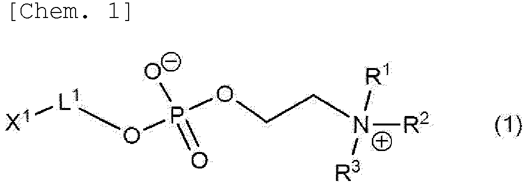 wherein
X¹ is a (meth)acryloyloxy group or a (meth)acryloylamino group,
L¹ is an alkylene group having 2 to 4 carbon atoms and optionally having one hydroxy group, or an alkyleneoxyalkylene group having 2 to 4 carbon atoms, and
R¹ to R³ are each independently an alkyl group having 1 to 3 carbon atoms.

2. The dry composition for nucleic acid amplification according to claim 1, wherein the polymer is a homopolymer consisting of one kind of constitutional unit derived from a monomer represented by the formula (1).

3. The dry composition for nucleic acid amplification according to claim 1, wherein the polymer is a copolymer further comprising a constitutional unit derived from a monomer represented by the formula (2): 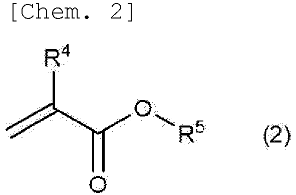 wherein
R⁴ is a hydrogen atom or a methyl group, and
R⁵ is a hydrogen atom or an alkyl group having 1 to 20 carbon atoms.

4. The dry composition for nucleic acid amplification according to claim 3, wherein the polymer is a copolymer further comprising a constitutional unit derived from a monomer represented by the formula (3): 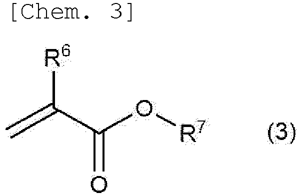 wherein
R⁶ is a hydrogen atom or a methyl group, and
R⁷ is an alkyl group having 3 to 6 carbon atoms, and two or more hydroxy groups.

5. The dry composition for nucleic acid amplification according to claim 1, wherein the polymer is a copolymer further comprising a constitutional unit derived from a monomer represented by the formula (4): 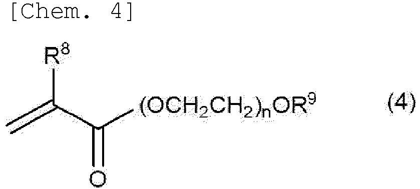 wherein
R⁸ is a hydrogen atom or a methyl group,
n is a number from 1 to 10, and
R⁹ is a hydrogen atom, a methyl group, or an ethyl group.

6. The dry composition for nucleic acid amplification according to any one of claims 1 to 5, further comprising a polymerase.

7. The dry composition for nucleic acid amplification according to claim 6, wherein the composition is used for a quantitative polymerase chain reaction method.

8. The dry composition for nucleic acid amplification according to claim 7, further comprising a primer.

9. A method for amplifying a nucleic acid, comprising preparing a reaction liquid by mixing the dry composition for nucleic acid amplification according to any one of claims 1 to 5, a nucleic acid to be amplified, and water, and
performing a nucleic acid amplification reaction using the obtained reaction liquid.
